# DEMANDE DE BREVET EUROPEEN

(11) **EP 2 290 598 A1**
(43) Date de publication de la demande: **02.03.2011**
(21) Numéro de dépôt: 10173907.6
(22) Date de dépôt: 24.08.2010
(51) Int. Cl.: G06Q 10/00, G06Q 50/00, G06F 19/00

(54) **Procédé de configuration d'un agenda électronique**

(30) Priorité: 31.08.2009 FR 0955917
(71) Demandeur: France Telecom, 75015 Paris (FR)
(72) Inventeur: Schiltz André, 38190, Bernin (FR); Peschet Pascal, 14160, Dives-sur-mer (FR); Hily, Serge, 14400, Bayeux (FR); Babin, Gerard, Bieville Beuville (FR)

(57) **Abrégé**

Ce procédé de configuration d'un agenda électronique embarqué dans un équipement électronique comporte :
- une étape (E30) d'obtention, à partir de données numériques résultant d'une numérisation d'une représentation d'un code, de données encodées dans ce code et comprenant au moins une information de date associée à un contenu; et
- une étape (E40) de configuration dudit agenda électronique pour programmer, à au moins une date ou heure définie par cette information de date, une exécution d'une fonction en rapport avec ledit contenu.

## Description

### Arrière-plan de l'invention

La présente invention permet la configuration automatique d'un équipement électronique.

Plus précisément, l'invention concerne un procédé pour configurer automatiquement un agenda électronique embarqué dans un équipement électronique.

Un équipement électronique au sens de l'invention peut notamment être constitué par un téléphone mobile, un assistant personnel, ou plus généralement par un équipement communiquant, par exemple portable.

L'invention trouve une application privilégiée mais non limitative pour aider un utilisateur à respecter une posologie, à savoir la fréquence et les doses de prises d'un médicament.

La société eMedicis propose sur son site Internet (http://www.e-medicis.com) un service pour rappeler à un utilisateur, par téléphone, messagerie ou courrier électronique, des moments de prises de médicaments et des rendez-vous médicaux.

Pour bénéficier de ce service, l'utilisateur doit se connecter à un site Internet, s'abonner, créer un profil personnel, et y saisir les informations de posologie pour planifier les dates et heures des rappels.

Malheureusement, ce service présente un inconvénient majeur en ce que la saisie des informations de posologie est effectuée par le patient lui-même, avec un risque d'erreur non négligeable, sans contrôle d'un pharmacien ou de tout autre personne qualifiée.

### Objet et résumé de l'invention

Selon un premier aspect, l'invention concerne un procédé de configuration d'un agenda électronique embarqué dans un équipement électronique, ce procédé comportant
- une étape d'obtention, à partir de données numériques résultant d'une numérisation d'une représentation d'un code, de données encodées dans ledit code et comprenant au moins une information de date associée à un contenu; et
- une étape de configuration dudit agenda électronique pour programmer, à au moins une date ou heure définie par ladite au moins une information de date, une exécution d'une fonction en rapport avec ledit contenu.

Corrélativement, l'invention vise aussi un équipement électronique comportant:
- un agenda électronique ;
- des moyens pour obtenir des données résultant d'une numérisation d'une représentation d'un code ;
- des moyens pour obtenir, à partir desdites données, au moins une information de date associée à un contenu ; et
- des moyens pour programmer dans ledit agenda électronique une exécution d'une fonction en rapport avec ledit contenu à au moins une date ou heure définie par ladite au moins une information de date.

Dans ce document, la notion "d'information de date" désigne une date au sens propre (jour, mois, année) mais aussi un horaire, ou une répétition de dates et/ou d'horaires.

Par exemple "tous les lundis à 8H15", "le premier mercredi de chaque mois" et "un jour sur deux" sont des "informations de date" au sens de l'invention.

La représentation du code est par exemple un code-barres (1D ou 2D) ou tout autre code graphique ou une représentation imprimable apte à encoder un code alphanumérique. On s'intéresse ici plus particulièrement à un code sous forme de séquence alphanumérique, pouvant facilement être représenté selon les modes de représentation qui viennent d'être cités.

Les données résultant de la numérisation peuvent être sous différentes formes selon le capteur utilisé pour les produire: une image dans le cas d'utilisation d'un capteur sous forme d'appareil photo ou, plus généralement, un signal numérique mono- ou bidimensionnel résultant du balayage par le capteur de la représentation du code.

La fonction dont l'exécution est programmée est choisie selon le champ d'application visé pour l'invention et les possibilités fonctionnelles de l'application mettant en oeuvre l'agenda électronique. A titre d'exemple de fonction, on peut citer :
- le déclenchement d'un rappel, sous forme de message textuel affiché automatique ou de message sonore déclenché automatiquement, en rapport avec ledit contenu;
- le déclenchement de l'exécution d'un script ou d'un programme prédéfini;
- un enregistrement de données, en rapport avec ledit contenu, dans l'agenda ou dans une mémoire de l'équipement électronique;
- un envoi de message, en rapport avec ledit contenu;
- un téléchargement de données, en rapport avec ledit contenu;
- une mise en communication;
- etc.

Une interface utilisateur de l'agenda électronique configuré par le procédé selon l'invention permettra donc une visualisation ou un paramétrage de la fonction dont l'exécution a été programmée. Un utilisateur pourra notamment vérifier / consulter / éditer les fonctions programmées et s'assurer de la cohérence de ces fonctions avec son emploi du temps. En particulier, un tel agenda électronique permet un affichage d'un calendrier présentant pour la date ou heure visée, des informations sur la fonction programmée et/ou le contenu et/ou les données encodées dans le code.

Dans une mise en oeuvre particulière, le procédé de configuration selon l'invention est utilisé pour mettre en place, au moyen de l'agenda électronique, un système de rappel automatique afin d'aider un patient à se conformer à une ordonnance médicale et à respecter une posologie indiquée par un médecin.

Dans cet exemple, des informations de prise de médicament correspondant à une ordonnance d'un médecin (typiquement un nom de médicament, une quantité, et un rythme de prise de ce médicament), peuvent être contrôlées par un pharmacien et saisies par ce dernier dans un système informatique apte à imprimer une représentation d'un code encodant ces informations. Dans ce cas, le code est utilisé pour encoder:
- d'une part, des informations de dates correspondant aux dates et/ou heures de prises de médicament ; et
- d'autre part, un contenu comprenant des informations de prise de médicament telles que nom du médicament, posologie, indications sur le mode d'absorption du médicament.

Dans cet exemple, le code peut alors être photographié par un appareil photographique intégré à un équipement électronique du patient, la photographie étant ensuite analysée afin d'en extraire le code et d'en déduire les données de prise de médicament, celles-ci étant utilisées pour configurer automatiquement l'agenda électronique du patient.

La configuration de l'agenda s'accompagne préférablement de la programmation d'un rappel pour la prise de médicament, par exemple au moyen d'alertes sonores ou de vibreur, aux dates et heures définies grâce aux informations de date extraites.

De façon très avantageuse, le patient n'intervient pas dans la saisie des données, ce qui évite tout risque d'erreur de sa part.

Conformément à l'invention, et au contraire du service proposé par la société eMedicis, la configuration de l'agenda électronique se fait hors ligne et est complètement automatisée, mis à part l'étape de saisie du code barre qui est déclenchée par un utilisateur.

En outre, l'utilisateur peut visualiser dans une interface utilisateur de son agenda électronique simultanément les rendez-vous qu'il a programmés au moyen de cet agenda électronique sur une période de temps donnée et les informations de prise de médicament relatives à cette même période de temps. Ceci est particulièrement intéressant pour une bonne organisation de l'emploi du temps de cet utilisateur.

Si cet utilisateur est par exemple amené à se déplacer pour ces rendez-vous, il pourra facilement organiser chaque journée pour emporter avec lui les médicaments à prendre pendant son déplacement. L'intégration d'informations de prise de médicament dans un agenda électronique est donc particulièrement avantageuse.

### Préférentiellement, le capteur est intégré à l'équipement électronique.

En variante, le capteur fait partie d'un autre dispositif apte à communiquer les données numériques produit de la numérisation ou les informations de date elles-mêmes à l'équipement électronique.

L'invention trouve des applications dans tout autre domaine d'activité dans lequel on souhaite configurer automatiquement l'agenda électronique d'un individu.

Dans un mode particulier de réalisation, les différentes étapes du procédé de configuration sont déterminées par des instructions de programme d'ordinateur, ce programme étant propre à être installé dans l'équipement communicant.

En conséquence, l'invention vise aussi un programme d'ordinateur, sur un support d'informations, ce deuxième programme comportant des instructions adaptées à la mise en oeuvre des étapes du procédé de configuration selon l'invention, ces étapes pouvant être mises en oeuvre par un équipement électronique ou plus généralement par un ordinateur.

Ce programme peut utiliser n'importe quel langage de programmation, et être sous la forme de code source, code objet, ou de code intermédiaire entre code source et code objet, tel que dans une forme partiellement compilée, ou dans n'importe quelle autre forme souhaitable.

L'invention vise aussi un support d'informations lisible par un ordinateur, et comportant des instructions d'un programme d'ordinateur tel que mentionné ci-dessus.

Le support d'informations peut être n'importe quelle entité ou dispositif capable de stocker le programme. Par exemple, le support peut comporter un moyen de stockage, tel qu'une ROM, par exemple un CD ROM ou une ROM de circuit microélectronique, ou encore un moyen d'enregistrement magnétique, par exemple une disquette (floppy disk) ou un disque dur.

D'autre part, le support d'informations peut être un support transmissible tel qu'un signal électrique ou optique, qui peut être acheminé via un câble électrique ou optique, par radio ou par d'autres moyens. Le programme selon l'invention peut être en particulier téléchargé sur un réseau de type Internet.

Alternativement, le support d'informations peut être un circuit intégré dans lequel le programme est incorporé, le circuit étant adapté pour exécuter ou pour être utilisé dans l'exécution du procédé en question.

### Brève description des dessins

D'autres caractéristiques et avantages de la présente invention ressortiront de la description faite ci-dessous, en référence aux dessins annexés qui en illustrent un exemple de réalisation dépourvu de tout caractère limitatif. Sur les figures :
- la figure 1 représente un système informatique d'un pharmacien ;
- la figure 2 représente de façon schématique, l'architecture matérielle d'un équipement électronique conforme à un mode particulier de réalisation de l'invention ; et
- la figure 3 représente, sous forme d'organigramme, les principales étapes d'un procédé de configuration conforme à un mode particulier de réalisation de l'invention.

### Description détaillée d'un mode de réalisation

Nous allons maintenant décrire une mise en oeuvre particulière de l'invention permettant d'aider un patient à respecter une posologie lors d'un traitement médical.

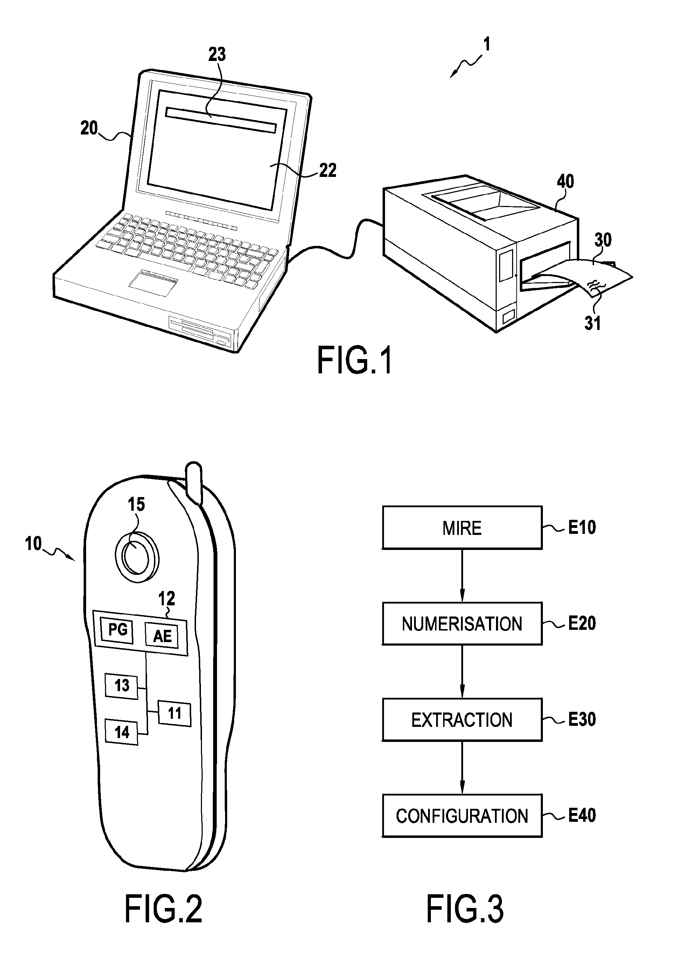

La figure 1 représente le système informatique 1 d'un pharmacien, ce système comportant principalement un ordinateur 20 apte à mettre en oeuvre une application logicielle 22 permettant la saisie de informations de prise de médicaments définies dans une ordonnance 30, et une imprimante 40 apte à imprimer une représentation d'un code encodant des informations de prise de médicaments. Cette impression peut s'effectuer sur l'ordonnance elle-même, sur la feuille de soin destinée à la sécurité sociale, ou sur tout autre support à partir duquel la représentation du code est susceptible d'être numérisée par un capteur.

Dans l'exemple de réalisation décrit ici, l'application logicielle 22 présente un champ de saisie 23 permettant au pharmacien de saisir des informations de prise de médicament pour un ou plusieurs médicaments prescrits par un médecin, à savoir dans cet exemple :
- le nom du médicament ;
- des indications sur le mode d'absorption du médicament (avec un verre d'eau, 15 mn avant le repas, etc);
- la posologie ou dose prescrite ; et
- des informations de date de prise du médicament.

A ce jour, ces données sont traditionnellement reportées par le pharmacien à la main sur les boites de médicaments.

Dans un mode de réalisation, l'application logicielle 22 propose au pharmacien une posologie standard, conforme à une recommandation du laboratoire fabricant du médicament, le pharmacien pouvant valider cette posologie ou l'ajuster pour un patient donné en fonction de la prescription médicale.

Dans le mode de réalisation décrit ici, l'application logicielle 22 génère ensuite un code-barres 31 encodant ces informations de prise de médicament, et imprime ce code-barres 31 sur l'ordonnance 30.

A titre d'exemple, le code-barres 31 est utilisé pour coder une séquence alphanumérique, par exemple sous la forme suivante:
#OS#Medicament: 100609:150609:10:14:18
Dans cette séquence alphanumérique :
- "OS" est un préfixe permettant de vérifier la validité du code-barres ;
- "Medicament" est le nom du médicament, avec ou sans la posologie, avec ou sans indications sur le mode d'absorption du médicament ;
- "100609" est la date de début du traitement ;
- "150609" est la date de fin du traitement ; et
- "10", "14" et "18" sont les heures de prises du médicament.

Tout autre mode de codage des informations de prise de médicament sous forme de séquence alphanumérique est également envisageable.

En variante, le code-barres peut contenir des informations complémentaires, par exemple, des effets indésirables susceptibles de se produire lors de la prise du médicament.

La figure 2 représente un équipement électronique 10 conforme à l'invention, à savoir, dans cet exemple un téléphone portable.

Dans l'exemple de réalisation décrit ici, le téléphone portable 10 a l'architecture matérielle d'un ordinateur. Il comporte notamment un processeur 11, une mémoire morte de type ROM 12, et une mémoire vive de type RAM 13.

La mémoire morte 12 de type ROM constitue un support d'enregistrement conforme à l'invention. Ce support d'enregistrement comporte un programme d'ordinateur PG, ce programme comportant des instructions pour exécuter un procédé de configuration conforme à l'invention et dont les principales étapes sont représentées à la figure 3.

La mémoire morte 12 de type ROM mémorise une application d'agenda électronique AE dont les données sont stockées dans la structure de données mémorisée dans une mémoire de type Flash 14. De manière connue, de telles données comprennent des données définissant les rendez-vous et/ou rappels et/ou tâches programmés dans l'agenda. Ces données comprennent notamment:
- date et heure du rendez-vous, du rappel ou de la tâche;
- fonction(s) à exécuter lors du rappel ou de la tâche;
- périodicité éventuelle du rendez-vous, du rappel ou de la tâche;
- message éventuellement associé au rendez-vous, au rappel ou à la tâche; etc.

De telles données sont modifiables soit à partir de l'agenda lui-même ou d'un module logiciel associé à cet agenda et chargé lors de l'exécution de cet agenda, soit à partir d'un module logiciel indépendant, apte à lire la structure de données de la mémoire Flash 14, apte à décoder puis interpréter ces données, apte à encoder les données modifiées dans le format utilisé pour leur stockage puis apte à stocker les données ainsi encodées dans la structure de données de la mémoire Flash 14.

Les instructions du programme informatique PG et celles de l'application AE d'agenda électronique peuvent être exécutées par le processeur 11, les variables nécessaires à l'exécution de ces programmes étant mémorisées dans la mémoire vive 13.

Le programme PG est apte à s'interfacer avec l'application d'agenda électronique ou est intégré dans une telle application d'agenda électronique ou partage avec cette application l'accès aux données de l'agenda électronique. Le programme PG est installé sur le téléphone portable 10 par téléchargement à partir d'un site Internet ou selon tout autre mode d'installation adéquat.

Quel que soit le mode de réalisation choisi pour le programme PG, ce programme est apte à configurer l'application d'agenda électronique AE c'est-à-dire à enregistrer des données de configuration dans la structure de données de la mémoire Flash 14.

Le téléphone portable 10 comporte aussi un appareil photographique 15 avec une résolution suffisante pour une acquisition d'une photo numérique du code-barres 31 permettant d'analyser les données numériques de la photo générée par l'appareil photographique 15 afin d'en extraire les informations de prise de médicament qui sont encodées dans ce code-barres 31.

Le programme PG est apte à obtenir les données numériques résultant de la numérisation du code-barres 31, c'est-à-dire la photo numérique du code-barres 31.

Dans l'exemple de réalisation décrit ici, le procédé de configuration selon l'invention comporte optionnellement une étape E10 de présentation d'une mire pour faciliter le positionnement du code-barres 31 par rapport au terminal mobile 10.

L'étape E10 est suivie par une étape E20 de numérisation du code-barres 31 imprimé, cette étape étant déclenchée par l'utilisateur.

L'étape E20 est suivie par une étape E30 d'obtention des informations de prise de médicament encodées dans le code-barres 31.

Cette étape E30 consiste à analyser les données numériques résultant de la numérisation du code-barres 31 (en l'occurrence, la photo numérique) afin d'en extraire la séquence alphanumérique représentée par ce code-barres 31, puis à interpréter cette séquence alphanumérique pour en extraire les informations de prise de médicament, c'est-à-dire les informations de date et le contenu associé à ces informations de date.

L'étape E30 est suivie par une étape E40 de configuration de l'application d'agenda électronique AE avec les informations de prise de médicament, cette étape consistant à enregistrer, dans la structure de données de la mémoire Flash 14, les informations de prise de médicament, notamment le contenu et les informations de date. Ces informations seront alors utilisables par l'agenda électronique AE.

Cette manière de procéder évite le développement d'une application dédiée à l'affichage, la consultation et l'édition des informations codées dans la représentation du code au moyen de l'interface utilisateur habituelle. En effet, une seule et même application d'agenda électronique permet l'affichage, la consultation et l'édition de la fonction programmée et/ou du contenu associé et/ou des informations codées dans la représentation du code (dans le code-barre) ainsi que l'affichage, la consultation et l'édition d'autres rendez-vous / rappels / tâches programmées par l'utilisateur au moyen de cet agenda électronique. Lorsque cet agenda électronique est mis en oeuvre dans un terminal à faibles ressources en mémoire, ceci constitue une économie précieuse des ressources disponibles.

L'étape E40 comprend en outre des opérations de configuration afin de programmer, à au moins une date ou heure définie par les informations de date, une exécution, à partir de l'agenda électronique, d'une fonction en rapport avec le contenu.

## Revendications

1. Procédé de configuration d'un agenda électronique (AE) embarqué dans un équipement électronique (10), ce procédé comportant:
- une étape (E30) d'obtention, à partir de données numériques résultant d'une numérisation d'une représentation (31) d'un code, de données encodées dans ledit code et comprenant au moins une information de date associée à un contenu; et
- une étape (E40) de configuration dudit agenda électronique (AE) pour programmer, à au moins une date ou heure définie par ladite au moins une information de date, une exécution d'une fonction en rapport avec ledit contenu.

2. Procédé de configuration selon la revendication 1 dans lequel l'étape de configuration comprend une étape d'enregistrement de ladite au moins une information de date et dudit contenu dans une structure de données de l'agenda électronique.

3. Procédé selon la revendication 1 comprenant une étape d'affichage d'un calendrier présentant pour ladite date ou heure des informations sur ladite fonction et/ou ledit contenu et/ou lesdites données.

4. Procédé de configuration selon la revendication 1 dans lequel ledit contenu comprend des informations relatives à une prise de médicament, ladite fonction étant un déclenchement d'un rappel pour ladite prise de médicament.

5. Procédé de configuration selon la revendication 1 dans lequel ladite représentation (31) est un code-barres.

6. Equipement électronique (10) comportant:
- un agenda électronique (AE) ; et
- des moyens (PG) pour obtenir des données résultant d'une numérisation d'une représentation (31) d'un code ;
ledit équipement étant **caractérisé en ce qu'**il comporte :
- des moyens (PG) pour obtenir, à partir desdites données, au moins une information de date associée à un contenu ; et
- des moyens (PG) pour programmer dans ledit agenda électronique une exécution d'une fonction en rapport avec ledit contenu à au moins une date ou heure définie par ladite au moins une information de date.

7. Equipement électronique selon la revendication 6 **caractérisé en ce qu'**il comprend un capteur apte à numériser ladite représentation.

8. Equipement électronique selon la revendication 6 ou 7 **caractérisé en ce que** ladite représentation est un code-barres.

9. Equipement électronique selon la revendication 6 comprenant des moyens d'enregistrement de ladite au moins une information de date et dudit contenu dans une structure de données de l'agenda électronique.

10. Equipement électronique selon la revendication 6 comprenant des moyens d'affichage d'un calendrier présentant des informations sur ladite fonction et/ou ledit contenu et/ou lesdites données.

11. Programme d'ordinateur (PG) comportant des instructions pour l'exécution du procédé de configuration selon l'une quelconque des revendications 1 à 5.

12. Support d'enregistrement lisible par un ordinateur sur lequel est enregistré un programme d'ordinateur (PG) comprenant des instructions pour l'exécution des étapes du procédé de configuration selon l'une quelconque des revendications 1 à 5.
